# EUROPEAN PATENT SPECIFICATION

(11) **EP 2 361 936 B1**
(45) Date of publication and mention of the grant of the patent: **20.04.2016**
(21) Application number: 10154751.1
(22) Date of filing: 25.02.2010
(51) Int. Cl.: C07K 16/46, C07K 16/18, C07K 16/28

(54) **Antigen-binding molecule and uses thereof**
Molécule à liaison d'antigène et ses utilisations
Antigenbindende Moleküle und ihre Verwendung

(43) Date of publication of application: 31.08.2011
(73) Proprietor: Affimed GmbH, 69120 Heidelberg (DE)
(72) Inventor: Little, Melvyn, 69151 Neckargemünd (DE); Le Gall, Fabrice, 68535 Edingen-Neckarhausen (DE)
(74) Representative: Schüssler, Andrea

(56) References cited:
- EP-A1- 1 500 665
- WO-A2-02/46232
- WO-A2-2006/125668
- KIPRIYANOV S M ET AL: "Bispecific tandem diabody for tumor therapy with improved antigen binding and pharmacokinetics" JOURNAL OF MOLECULAR BIOLOGY, LONDON, GB LNKD- DOI:10.1006/JMBI.1999.3156, vol. 293, no. 1, 15 October 1999 (1999-10-15), pages 41-56, XP004457324 ISSN: 0022-2836
- KIPRIYANOV SERGEY M: "Generation of bispecific and tandem diabodies" METHODS IN MOLECULAR BIOLOGY, HUMANA PRESS INC, NJ, US, vol. 562, 1 January 2009 (2009-01-01), pages 177-193, XP009134829 ISSN: 1064-3745
- COCHLOVIUS BJOERN ET AL: "Cure of Burkitt's lymphoma in severe combined immunodeficiency mice by T cells, tetravalent CD3 X CD19 tandem diabody, and CD28 costimulation" CANCER RESEARCH, vol. 60, no. 16, 15 August 2000 (2000-08-15), pages 4336-4341, XP002587518 ISSN: 0008-5472
- LE GALL FABRICE ET AL: "Effect of linker sequences between the antibody variable domains on the formation, stability and biological activity of a bispecific tandem diabody" PROTEIN ENGINEERING, DESIGN AND SELECTION, OXFORD JOURNAL, LONDON, GB LNKD- DOI:10.1093/PROTEIN/GZH039, vol. 17, no. 4, 1 April 2004 (2004-04-01), pages 357-366, XP002429042 ISSN: 1741-0126
- ARNDT M A E ET AL: "Antigen binding and stability properties of non-covalently linked anti-CD22 single-chain Fv dimers" FEBS LETTERS, ELSEVIER, AMSTERDAM, NL LNKD- DOI:10.1016/J.FEBSLET.2004.11.011, vol. 578, no. 3, 17 December 2004 (2004-12-17), pages 257-261, XP004675824 ISSN: 0014-5793
- DESPLANCQ D ET AL: "MULTIMERIZATION BEHAVIOUR OF SINGLE CHAIN FV VARIANTS FOR THE TUMOUR-BINDING ANTIBODY B72.3" PROTEIN ENGINEERING, OXFORD UNIVERSITY PRESS, SURREY, GB, vol. 7, no. 8, 1 August 1994 (1994-08-01), pages 1027-1033, XP000445077 ISSN: 0269-2139
- LU D ET AL: "The effect of variable domain orientation and arrangement on the antigen-binding activity of a recombinant human bispecific diabody" BIOCHEMICAL AND BIOPHYSICAL RESEARCH COMMUNICATIONS, ACADEMIC PRESS INC. ORLANDO, FL, US LNKD- DOI:10.1016/J.BBRC.2004.04.060, vol. 318, no. 2, 28 May 2004 (2004-05-28), pages 507-513, XP004505410 ISSN: 0006-291X
- R. ASANO ET AL: "Cytotoxic Enhancement of a Bispecific Diabody by Format Conversion to Tandem Single-chain Variable Fragment (taFv): THE CASE OF THE hEx3 DIABODY", JOURNAL OF BIOLOGICAL CHEMISTRY, vol. 286, no. 3, 21 January 2011 (2011-01-21), pages 1812-1818, XP055043825, ISSN: 0021-9258, DOI: 10.1074/jbc.M110.172957

## Description

### Background of the invention

Various formats of multivalent recombinant antibody fragments have been designed as alternatives to quadroma derived antibodies.

US 7,129,330 and Kipriyanov et al. J. Mol. Biol. (1999) 293, 41- 56 describe the construction and production of a particular format of multivalent antibody fragments which are named "tandem diabodies" (TandAb^{®}), since their design is based on intermolecular pairing of V_{H} and V_{L} variable domains of two different polypeptides as described for diabodies (Holliger et al.,1993, Proc. Natl. Acad. Sci. USA, 90:6444-6448). The described antibodies are bispecific for CD19 and CD3. In contrast to bivalent scFv-scFv (scFv)₂ tandems the tandem diabodies are tetravalent, because they have four antigen-binding sites. Polypeptides with the domain order V_{H}A-V_{L}B-V_{H}B-V_{L}A from the N-terminus to the C-terminus of the polypeptides forming the tandem diabodies are described. The orders of variable domains and the linker peptides between them were designed such that each domain associates with a complementary domain in another identical molecule thereby forming the dimerized tetravalent tandem diabodies. The tandem diabodies are devoid of immunoglobulin constant domains. It was reported that the tandem diabodies have advantages such as a high affinity, a higher avidity, lower clearance rates and exhibit a favorable in vitro and in vivo efficiency.

Several additional tandem diabodies are known comprising antibody specificities such as, for example, anti-CD16, anti-EpCAM and anti-CD30. In all cases, however, the order of the four antibody domains along the polypeptide chains of the tandem diabody from the N-terminus to the C-terminus was always V_{H}A-V_{L}B-V_{H}B-V_{L}A, where V_{H} and V_{L} represent the antibody heavy and light chain variable domains of antibodies with specificities for antigens A and B, respectively.

Such bispecific tandem diabodies can make a bridge between a tumor cell (e.g. B-CLL cell) and an effector cell of the human immune system (NK cell, T cell, monocyte, macrophage or granulocyte) thus permitting killing of the tumour cell. The tight binding of the tumor cell and the cytotoxic cell induces the destruction of the tumor cell.

While such tandem diabodies have proved to be favorable for therapeutic applications, e.g. for therapeutic concepts for the treatment of tumors, there remains a need for improved antigen-binding molecules for enhancing the immune response.

Le Gall F, et al.(Protein Engineering, Design and Selection, 2004, 17(4):257-261) reports the effect of different linker lengths on the activity of a bispecific CD3xCD19 tandem diabody with the variable domains in the order V_{H}CD3-V_{L}CD19-V_{H}CD19-V_{L}CD3.

### BRIEF DESCRIPTION OF THE DRAWINGS

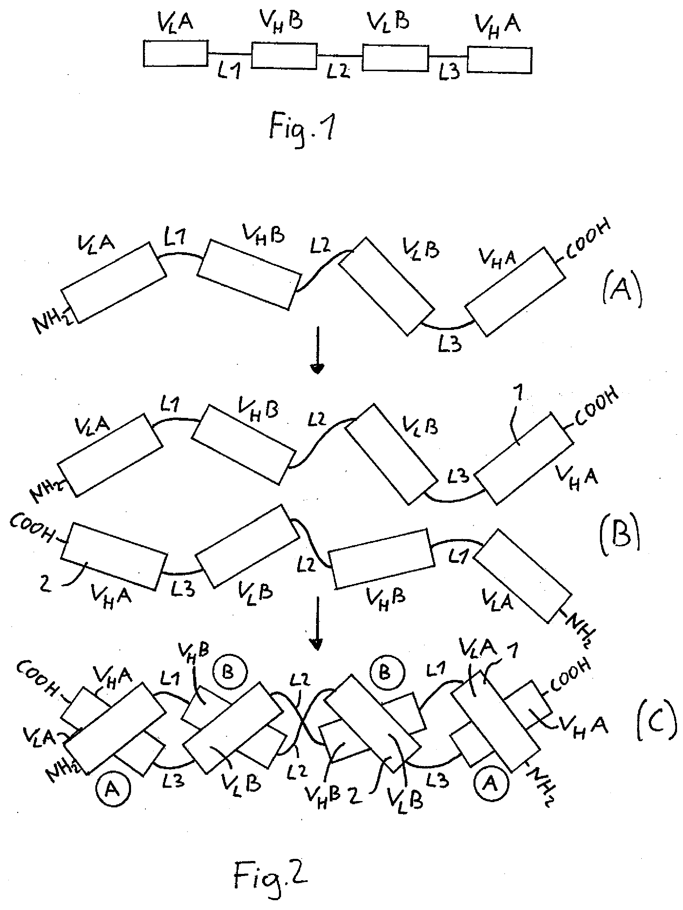

- Fig. 1: illustrates the gene organization of a construct encoding an antigen-molecule according to the invention, where V_{L}A represents a light chain variable immunoglobulin domain specific for an antigen A, V_{H}B represents a heavy chain variable immunoglobulin domain specific for an antigen B, V_{L}B represents a light chain variable immunoglobulin domain specific for the antigen B, V_{H}A represents a heavy chain variable immunoglobulin domain specific for the antigen A, L1 a peptide linker or a peptide bond connecting V_{L}A and V_{H}B, L2 a peptide linker or a peptide bond connecting V_{H}B and V_{L}B, and L3 a peptide linker or a peptide bond connecting V_{L}B and V_{H}A.
- Fig. 2: illustrates the formation of a dimeric antigen-binding molecule according to the invention from non-functional monomeric polypeptide chains (A) by intra-molecular pairing of variable domains of a first polypeptide chain 1 and a second polypeptide chain 2 with one another (B) to a functional antigen-binding molecule according to the inventions in the format of a tandem diabody, where "1" represents the first polypeptide chain, "2" represents the second polypeptide chain, V_{L}A represents a light chain variable immunoglobulin domain specific for an antigen A, V_{H}B represents a heavy chain variable immunoglobulin domain specific for an antigen B, V_{L}B represents a light chain variable immunoglobulin domain specific for the antigen B, V_{H}A represents a heavy chain variable immunoglobulin domain specific for the antigen A, L1 a peptide linker or a peptide bond connecting V_{L}A and V_{H}B, L2 a peptide linker or a peptide bond connecting V_{H}B and V_{L}B, and L3 a peptide linker or a peptide bond connecting V_{L}B and V_{H}A

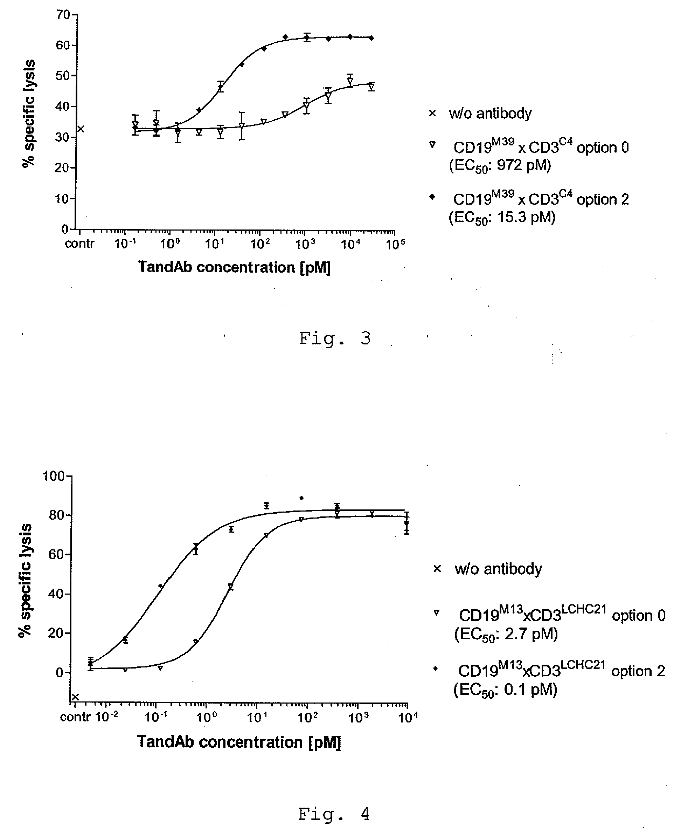

- Fig. 3: shows a comparison of CD19xCD3 tandem diabodies in a cytotoxicity assay. Option 0 = antibody A1 with the domain order V_{H}A-V_{L}B-V_{H}B-V_{L}A. Option 2 = antibody B with the domain order V_{L}A-V_{H}B-V_{L}B-V_{H}A according to the invention. 1x10⁴ calcein-labelled Raji cells were incubated with 5x10⁵ PBMC in the presence of increasing concentrations of the indicated CD19xCD3 tandem diabodies. PBMC were cultured overnight in the presence of 25 U/mL human IL-2 before they were used as effector cells in the assay. After 4 h incubation fluorescent calcein in the cell culture medium released from apoptotic target cells was measured at 520 nm and % specific lysis was calculated. EC₅₀ values were analysed by non-linear regression using GraphPad soft-ware. The mean and standard deviations of duplicates were plotted.
- Fig. 4: shows a comparison of CD19xCD3 tandem diabodies in a cytotoxicity assay. Option 0 = antibody A2 with the domain order V_{H}A-V_{L}B-V_{H}B-V_{L}A. Option 2 = antibody C with the domain order V_{L}A-V_{H}B-V_{L}B-V_{H}A according to the invention. 1x10⁴ calcein-labelled Raji cells were incubated with 5x10⁵ freshly isolated PBMC in the presence of increasing concentrations of the indicated CD19xCD3 tandem diabodies. After 4 h incubation fluorescent calcein in the cell culture medium released from apoptotic target cells was measured at 520 nm and % specific lysis was calculated. EC₅₀ values were analysed by non-linear regression using GraphPad software. The mean and standard deviations of duplicates were plotted.

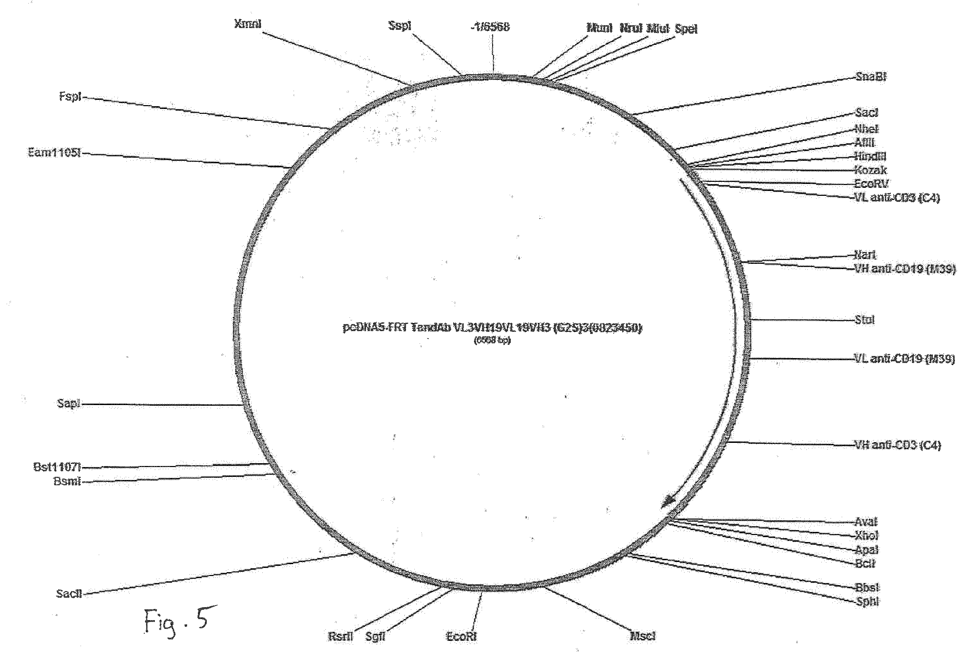

- Fig. 5: shows the vector map with the restriction sites of pCDNA5FRT which encodes antibody B.VH and VL: variable domains of the heavy and the light chains.

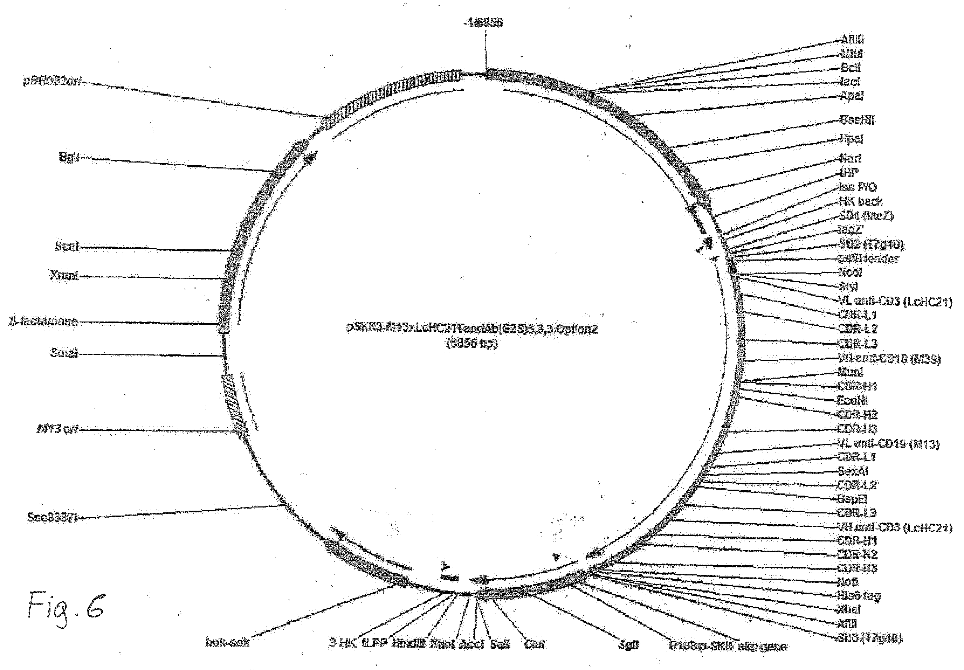

- Fig. 6: shows the vector map with the restrictions sites of pSKK3 which encodes antibody C. VH and VL: variable domains of the heavy and light chains.

### Detailed Description of the Invention

The present invention provides a recombinant dimeric and tetravalent antigen-binding molecule with four immunoglobulin domains (two heavy chain variable domains and two light chain variable domains) linked with one another in a polypeptide chain and arranged in the order V_{L}A-V_{H}B-V_{L}B-V_{H}A from the N-terminus to the C-terminus of the polypeptide chain and being specific for CD3 and CD19. Such an antigen-binding molecule of the present invention triggers an enhanced immune response.

In one embodiment, it illustrates that a dimeric, bispecific antigen-binding molecule of the tandem diabody format being specific for CD3 and CD19 and having polypeptide chains with the domain order V_{L}A-V_{H}B-V_{L}B-V_{H}A is more than 6o times more active *in vitro,* i.e. cytotoxic, than a corresponding tandem diabody molecule with the same domains but in the reverse domain order V_{H}A-V_{L}B-V_{H}B-V_{L}A.

Thus, tandem diabodies with the domain order V_{L}A-V_{H}B-V_{L}B-V_{H}A from the N-terminus to the C-terminus of the polypeptide chains have an increased potential for immunotherapy. A further advantage of the enhanced cytotoxic activity is that the effective therapeutic dosages for such tandem diabodies may be reduced. Moreover, side effects caused by the administered antigen binding molecules may also be reduced due to the lower dosages. Without being bound by any theory, the new domain order allows a modified crosslinking of the dimeric antigen binding molecule between CD3 as the antigen A and CD19 as the antigen B compared with the tandem diabodies of the art and, in certain aspects of the invention, this will enable the molecule to bind to the target antigens more efficiently than the dimeric antigen binding molecules of the art.

Therefore, the biological activity of a tandem diabody can be enhanced, when the four variable domains of each polypeptide chain which form the dimeric antigen-binding molecule are arranged in the order V_{L}A-V_{H}B-V_{L}B-V_{H}A from the N-terminus to the C-terminus of each polypeptide chain.

The present invention provides a dimeric antigen-binding molecule comprising a first and a second polypeptide chain, wherein each of the first and the second polypeptide chains comprises a first domain V_{L}A being a light chain variable domain specific for a first antigen A, a second domain V_{H}B being a heavy chain variable domain specific for a second antigen B, a third domain V_{L}B being a light chain variable domain specific for the second antigen B, a fourth domain V_{H}A being a heavy chain variable domain specific for the first antigen A, and said domains are arranged in each of said first and second polypeptide chains in the order V_{L}A-V_{H}B-V_{L}B-V_{H}A from the N-terminus to the C-terminus of said polypeptide chains, wherein antigen A is CD3 and antigen B is CD19.

The first, second, third and fourth variable domains are arranged in an orientation preventing intramolecular pairing within the same polypeptide chain and the first polypeptide chain is associated, i.e. dimerized, with the second polypeptide chain such that the first domain V_{L}A of the first polypeptide chain is in association with the fourth domain V_{H}A of the second polypeptide chain to form an antigen binding site for the first antigen A, the second domain V_{H}B of the first polypeptide chain is in association with the third domain V_{L}B of the second polypeptide chain to form an antigen binding site for the second antigen B, the third domain V_{L}B of the first polypeptide chain is in association with the second domain V_{H}B of the second polypeptide chain to form an antigen binding site for the second antigen B and the fourth domain V_{H}A of the first polypeptide chain is in association with the first domain V_{L}A of the second polypeptide chain to form an antigen binding site for the first antigen A.

The term "antigen-binding molecule" refers to an immunoglobulin derivative with multivalent antigen-binding properties, having at least four antigen-binding sites. Each antigen-binding site is formed by a heavy chain variable domain V_{H} and a light chain variable domain V_{L} of the same antigen, i.e. epitope, specificity. Preferably the antigen-binding molecule according to the invention is devoid of immunoglobulin constant domains or fragments of immunoglobulin constant domains, but in certain cases described below a constant domain or parts thereof may be linked to the antigen-binding molecule.

The antigen-binding molecule is "dimeric" which term refers to a complex of two polypeptide monomers. These two polypeptide monomers are the first and the second polypeptide chains. Preferably the antigen-binding molecule is a "homodimer" which term means that the antigen-binding molecule is composed of identical polypeptide monomers. In a preferred homodimeric antigen-binding molecule according to the invention the first and the second polypeptide chain may have the same amino acid sequence, i.e. the first and the second polypeptide chains are identical and, thus, are encoded and expressed by the same single polynucleotide. This is different in the case of so-called bispecific diabodies, which are heterodimers that are encoded by two distinct polynucleotides. In the former case each of the first and the second polypeptide chains contain four variable domains, four binding sites are formed and the antigen-binding molecule is tetravalent. Such tetravalent homodimeric antigen-binding molecules have received some recognition in the art as tandem diabodies.

Preferably, in the antigen-binding molecule the first and the second polypeptide chain are non-covalently associated with each other, in particular with the proviso that there is no covalent bound between the first and second polypeptide chain. However, if desired, the two polypeptide chains may be additionally stabilized by at least one covalent linkage, e.g. by a disulfide bridge between cysteine residues of different polypeptide chains.

The term "polypeptide chain" refers to a polymer of amino acid residues linked by amide bonds. The first and the second polypeptide chains are, preferably, single chain fusion proteins which are not branched. In each of the first and second polypeptide chains the four domains are arranged such that the second domain V_{H}B is C-terminal from the first domain V_{L}A, the third domain V_{L}B is C-terminal from the second domain V_{H}B and the fourth domain V_{H}A is C-terminal from the third domain V_{L}B. The first and the second polypeptide chains may have contiguous amino acid residues in addition N-terminal to the first domain V_{L}A and/or C-terminal to the fourth domain V_{H}A. For example, the polypeptide chain may contain a Tag sequence, preferably at the C-terminus which might be useful for the purification of the polypeptide. An example of a Tag sequence is a His-Tag, e.g. a His-Tag consisting of six His-residues.

In some embodiments, the first, second, third and fourth domains are covalently connected such that the domains of the same polypeptide chain do not associate, i.e. pair, with each other. The domains may be linked such that the first domain V_{L}A is linked with the second domain V_{H}B by a first linker L1, the second domain V_{H}B is linked with the third domain V_{L}B by a second linker L2 and the third domain V_{L}B is linked with the fourth domain V_{H}A by a third linker L3, wherein the first linker L1 and the third linker L3 are distal to the central linker L2 on each of the first and second polypeptide chains..

The length of each of the linkers L1, L2 and L3 is such that the domains of the first polypeptide chain can associate with the domains of the second polypeptide chain to form the dimeric antigen-binding molecule. The length of the linkers influences the flexibility of the antigen-binding molecule. The desired flexibility of the antigen-binding molecule depends on the target antigen density and the acessibility of the target antigen, i.e. epitopes. Longer linkers provide more flexible antigen-binding molecules with more agile antigen-binding sites. The effect of linker length on the formation of dimeric antigen-binding molecules is described, for example, in Todorovska et al., 2001 Journal of Immunological Methods 248:47-66; Perisic et al., 1994 Structure 2:1217-1226; Le Gall et al., 2004, Protein Engineering 17:357-366 and WO 94/13804.

The linkers L1, L2 and/or L3 are "short", i.e. consist of 6, 7, 8, 9, 10, 11 or 12 amino acid residues. Such short linkers favor the correct dimerization of the first with the second polypeptide chain by binding and forming antigen-binding sites between light chain variable domains and heavy chain variable domains of different polypeptide chains. In particular, the central linker L2 should be short such that it prevents formation of a single chain Fv (scFv) antigen-binding unit within the same polypeptide chain by the two adjacent domains V_{H}B and V_{L}B. The central linker L2 influences the flexibility of the polypeptide chain. If the central linker L2 is long, and flexible (in general consisting of about 12 or more amino acid residues) the polypeptide chain can fold head-to-tail and form a single-chain antigen-binding molecule known in the art as a single chain diabody. If the central linker L2 is short and rigid the polypeptide chain cannot fold head-to-tail and dimerizes with another polypeptide chain. Shortening the linker to about 12 or less amino acid residues generally prevents adjacent domains of the same polypeptide chain from interacting with each other. Therefore, the central linker L2 and the distal linkers L1 and L3 should consist of 6- 12 amino acid residues to prevent pairing of adjacent domains of the same polypeptide chain. The linkers may consist of different numbers of amino acid residues, but it is preferred that the distal linkers L1 and L3 have the same number of amino acid residues or do not differ in length by more than one or two amino acid residues. In a certain aspect of the invention at least one of the linkers L1, L2 and/or L3 consists of nine amino acid residues. In a particular embodiment of the invention all three linkers L1, L2 and L3 consist of nine amino acid residues.

Regarding the amino acid composition of the linkers, in some embodiments, peptides are selected that do not interfere with the dimerization of the first and second polypeptide chains. For example, linkers comprising glycine and serine residues generally provide flexibility and protease resistance. The amino acid sequence of the linkers can be optimized, for example, by phage-display methods to improve the antigen binding and production yield of the molecules. In particular embodiments of the invention the linker may comprise the amino acid sequence GGSGGSGGS.

The first domain V_{L}A, the second domain V_{H}B, the third domain V_{L}B and the fourth domain V_{H}A are light chain and heavy chain variable domains of an immunoglobulin. The variable domains comprise the hypervariable loops or complementary binding regions (CDRs) containing the residues in contact with the antigen and the segments which contribute to the correct folding and display of the CDRs. It is preferred that each of the heavy chain and light chain variable domains comprises the respective three CDRs. The domains may be derived from any immunoglobulin class, e.g., IgA, IgD, IgE and IgM or a subclass thereof. The immunoglobulin may be of animal, in particular mammal, origin. Each domain may be a complete immunoglobulin heavy or light chain variable domain, a mutant, fragment or derivative of a naturally occurring variable domain, or a synthetic, e.g. recombinant domain which is genetically engineered. A derivative is a variable domain which differs by the deletion, substitution, addition or insertion of at least one amino acid from the amino acid sequence of a naturally occurring variable domain. Synthetic, e.g. recombinant domains, can be obtained, for example, by well known reproducible methods from hybridoma-derived antibodies or phage-display immunoglobulin libraries. For example phage display methods can be used to obtain variable domains of human antibodies to an antigen by screening libraries from human immunoglobulin sequences. The affinity of initially selected antibodies can be further increased by affinity maturation, for example chain shuffling or random mutagenesis. A person of ordinary skill in the art is familiar with methods for obtaining domains from natural or recombinant antibodies (for laboratory manuals see, for example, Antibody engineering: methods and protocols / edited by Benny K.C. Lo; Benny K.C. II Series: Methods in molecular biology (Totowa, N.J.)).

In a certain aspect of the invention at least one, preferably all, of the first domain V_{L}A, the second domain V_{H}B, the third domain V_{L}B and the fourth domain V_{H}A are fully human, humanized or chimeric domains. A humanized variable domain comprises a framework region substantially having the amino acid sequence of a human immunoglobulin and a CDR of a non-human immunoglobulin. Humanized antibodies can be produced by well-established methods such as, for example CDR-grafting (see, for example, Antibody engineering: methods and protocols / edited by Benny K.C. Lo; Benny K.C. II Series: Methods in molecular biology (Totowa, N.J.)). Thus, a skilled person is readily able to make a humanized or fully human version of antigen-binding molecules and variable domains from non-human, e.g. murine, sources with the standard molecular biological techniques known in the art for reducing the immunogenicity and improving the efficiency of the antigen-binding molecule in a human immune system. In a preferred embodiment of the invention all domains (e.g. V_{L}A, V_{H}B, V_{L}B and V_{H}A) are humanized or fully human; most preferred, the dimeric antigen-binding molecule according to the invention is humanized or fully human. The term "Fully human" as used herein means that the amino acid sequences of the variable domains and the peptides linking the variable domains in the first and second polypeptide chains originate or can be found in humans. In certain embodiments of the invention the variable domains may be human or humanized but not the peptides linking the variable domains.

CD3 antigen is associated with the T-cell receptor complex on T-cells. The binding of the dimeric antigen-binding molecule according to the invention to CD3 triggers the cytotoxic activity of T-cells. By bispecific binding of the dimeric antigen binding molecule to CD3 and to a target cell, e.g. tumor cell, cell lysis of the target cell may be induced. Dimeric antigen-binding molecules with a specificity towards CD3 and their production are known in the art (and described for example in Kipriyanov et al., 1999, Journal of Molecular Biology 293:41-56, Le Gall et al., 2004, Protein Engineering, Design & Selection, 17/4:357-366)

Dimeric antigen-binding molecules according to the invention, wherein the tumor specificity is towards CD19 antigen may be used for immunotherapy of B-cell malignancies, because the CD19 antigen is expressed on virtually all B-lineage malignancies from lymphoblastic leukemia (ALL) to non-Hodgkin's lymphoma (NHL). In particular for the treatment of non-Hodgkin's lymphoma dimeric antigen-binding molecules having specificity towards CD19 can be used. Dimeric antigen-binding molecules having specificity towards CD19 and their production are known in the art (and described, for example, in Cochlovius et al., 2000, Cancer Research 60:4336-4341).

The antigen binding molecule as described herein is dimeric and bispecific for CD3 and CD19.

The first domain V_{L}A and the fourth domain V_{H}A are specific for CD3, while the second domain V_{H}B and the third domain V_{L}B are specific for CD19. The first and second polypeptide chains each have the domain order V_{L}^{CD3}-V_{H}^{CD19}- V_{L}^{CD19}-V_{H}^{CD3} from the N-terminus to the C-terminus of the polypeptide chains. In a preferred embodiment the first, second, third and fourth domains are humanized or fully human. In a most preferred embodiment the first and second polypeptide chain as defined above is humanized or fully human.

A further aspect of the invention provides a dimeric antigen-binding molecule according to any one of the embodiments described above which is linked with a further functional unit, e.g. a functional domain or agent, which independently mediates a biological function, in particular a biochemical event. The further functional unit may be complexed with or covalently bound to at least one of the two individual polypeptide chains of the dimeric antigen-binding molecule. In one aspect, the further functional unit may be covalently bound to only one of the individual polypeptide chains and in another aspect the further functional unit may be covalently bound to both polypeptide chains of the dimeric antigen-binding molecule thereby linking the two polypeptide chains. In a further aspect, each of the two polypeptide chains is covalently bound individually to a further functional unit. When the further functional unit is covalently bound to at least one of the two polypeptide chains, the further functional unit may be fused to at least one of the two polypeptide chains by a peptide bond or a peptide linker. Alternatively, the further functional unit may be linked by a chemical conjugation such as a disulfide bridge, e.g. between a cysteine residue of at least one polypeptide chain and a cysteine residue of the further functional unit, ester linkage or by chemical crosslinking. In a certain aspect of the invention the further functional unit may be linked to the antigen binding molecule by a cleavable linker such as, for example, a disulfide bound.

The further functional unit may be linked to the N-terminus or C-terminus of the first and/or second polypeptide chains. If one further functional unit is linked to both, the first and second, polypeptide chains, the further functional unit may be linked N-terminal to one polypeptide chain and C-terminal to the other polypeptide chain.

Homobifunctional and heterobifunctional reagents for chemical crosslinking of a polypeptide chain with a further functional unit such as a further polypeptide or an agent are well known in the art. Examples include but are not limited to 5,5'-dithiobis(2-nitrobenzoic acid) (DTNB), o-phenylenedimaleimide (o-PDM), succinimidyl 3-(2-pyridyldithio)propionate (SPDP), N-succinimidyl S-acetylthio acetate (SATA), succinimidyl 4-(N-maleimidomethyl)cyclohexane-1-carboxylate (SMCC) or 4-(4-N-maleimidophenyl)butyric acid hydrazide (MPBH). Methods for crosslinking of polypeptide chains comprising immunoglobulin chains with a further polypeptide or a chemical agent are described for example in Graziano et al., Methods in Molecular Biology, 2004, vol. 283, 71-85 and Hermanson, G.T. "Bioconjugate Techniques" Academic Press, London 1996.

In one aspect the further functional unit may be at least one further variable immunoglobulin domain. The further variable immunoglobulin domain may be specific for the first antigen A or the second antigen B for which the binding sites of the dimeric antigen-binding molecule are specific or, alternatively, specific for a third antigen C which is different from antigen A and antigen B. In a certain aspect a further light chain variable domain V_{L} and a further heavy chain variable V_{H} may be fused to each of the two polypeptide chains such that one further domain, in particular V_{H}, is fused to the N-terminus and the other further domain, in particular V_{L}, is fused to the C-terminus resulting in a polypeptide having six variable domains which will associate with another identical polypeptide to a dimeric antigen-binding molecule having six antigen-binding sites. In another aspect one further variable immunoglobulin domain may be fused to one of the polypeptide chains of the antigen-binding molecule which then non-covalently associates with a complementary variable immunoglobulin domain with the same specificity of a further third polypeptide thereby forming a further antigen-binding site between the dimeric antigen-binding molecule and the further third polypeptide. In another aspect a further antigen-binding unit including a scFv or a diabody may be linked as a further functional unit to the dimeric antigen-binding molecule.

In a certain aspect the further functional unit may be at least one further dimeric antigen-binding molecule as described herein. Accordingly, two or more dimeric antigen-binding molecules according to the invention may be linked with one another to increase the valency and avidity of the antigen binding molecules.

In another aspect the further functional unit may be an effector domain including Fc domain, CH2 domain, CH3 domain, hinge domain or a fragment thereof. Such a unit may confer effector properties on the antigen-binding molecule in the case of binding to Fc receptors. Such functional units may further be used to increase the serum-half life of the antigen-binding molecule.

In another aspect the further functional unit may be an enzyme. In the case where the enzyme is capable of converting a pro-drug to an active drug, such an antigen-binding molecule may be used in antibody-dependent enzyme prodrug therapy (ADEPT). For this the antigen-binding molecule directs the enzyme to the tissue of interest and when the antigen-binding molecule binds to the tissue, the prodrug is activated at that site.

In another aspect the functional unit may be a drug, toxin, radioisotope, lymphokine, chemokine or labeling molecule. Such an antigen-binding molecule delivers the functional unit to the desired site of action. For example a chemotherapeutic drug linked to an antigen-binding molecule being specific for a tumor antigen can be delivered to a tumor cell and toxins may be delivered to pathogens or tumor cells. Examples of a toxin are but not limited to ribosyl transferase, serine protease, guanyl cyclase activator, calmodulin dependent adenyl cyclase, ribunuclease, DNA alkylating agent or mitosis inhibitor, e.g. doxorubicin. The labeling molecule may be, for example, a fluorescent, luminescent or radiolabel molecule, a metal chelate or an enzyme (e.g. horse-radish peroxidase, alkaline phosphatase, ß-galactosidase, malate dehydrogenase, glucose oxidase, urease, catalase etc.) which, in turn, when later exposed to a substrate will react to the substrate in such a manner as to produce a chemical moiety which can be detected and can be used for *in vivo* imaging or immunoassays, when it is linked to the antigen-binding molecule according to the invention. When used for an immunoassay, the dimeric antigen-binding molecule can also be immobilized on an insoluble carrier, e.g. glass, polystyrene, polypropylene, polyethylene, dextran, nylon, natural and modified celluloses, polyacrylamides, agarose and magnetic beads.

For increasing serum-half life of the antigen-binding molecules according to the invention in the body, the antigen-binding molecule, if desired, may be fused to albumin or pegylated, sialylated or glycosylated (see, for example, Stork et al., 2008, J. Biol. Chem., 283:7804-7812).

The dimeric antigen-binding molecule according to any one of the embodiments described here previously may be produced by expressing polynucleotides encoding the individual polypeptide chains which associate with each other to form the dimeric antigen-binding molecule. Therefore, a further embodiment of the invention are polynucleotides, e.g. DNA or RNA, encoding the polypeptide chains of the dimeric antigen-binding molecule as described herein above.

The polynucleotides may be constructed by methods known to the skilled person, e.g. by combining the genes encoding the first domain V_{L}A, the second domain V_{H}B, the third domain V_{L}B and the fourth domain V_{H}A either separated by peptide linkers or directly linked by a peptide bound, into a single genetic construct operably linked to a suitable promoter, and optionally a suitable transcription terminator, and expressing it in bacteria or other appropriate expression system. Depending on the vector system and host utilized, any number of suitable transcription and translation elements, including constitutive and inducible promoters, may be used. The promoter is selected such that it drives the expression of the polynucleotide in the respective host cell.

The polynucleotides may be codon optimized with the codon bias being altered to suit the particular expression in the chosen host.

The polynucleotide may be inserted into vectors, preferably expression vectors, which represent a further embodiment of the invention. These recombinant vectors can be constructed according to methods well known to the person skilled in the art; see, e.g., Sambrook, Molecular Cloning A Laboratory Manual, Cold Spring Harbor Laboratory (1989) N.Y.

A variety of expression vector/host systems may be utilized to contain and express the polynucleotides encoding the polypeptide chains of the present invention. These include, but are not limited to, microorganisms such as bacteria transformed with recombinant bacteriophage, plasmid, or cosmid DNA expression vectors, yeast transformed with yeast expression vectors; insect cell systems infected with virus expression vectors (e.g., baculovirus); plant cell systems transformed with virus expression vectors (e.g., cauliflower mosaic virus, CaMV; tobacco mosaic virus, TMV) or with bacterial expression vectors (e.g., Ti or pBR322 plasmids); or animal cell systems, for which, e.g., viral-based expression systems may be utilised.

A particular preferred expression vector for expression in *E.coli* is pSKK (LeGall et al., J Immunol Methods. (2004) 285(1):111-27) or pcDNA5 (Invitrogen) for the expression in mammal cells.

Thus, the dimeric antigen-binding molecule as described herein may be produced by introducing a polynucleotide or vector encoding the polypeptide chain as described above into a host cell and culturing said host cell under conditions whereby the polypeptide chain is expressed. The dimeric antigen-binding molecule obtained from the expressed polypeptide chains may be isolated and, optionally, further purified. Conditions for the growth and maintenance of host cells, the expression, isolation and purification of dimeric antigen-binding molecules according to the invention from these host cells are fully described in the art.

In a further embodiment of the invention compositions comprising a dimeric antigen-binding molecule or a polynucleotide as described herein above and at least one further component are provided. For use in preventing or treating a disease or disorder the composition containing the dimeric antigen-binding molecule or the polynucleic acid molecule encoding the polypeptide chains forming the antigen-binding molecule is preferably combined with a suitable pharmaceutically acceptable carrier. The term "pharmaceutically acceptable carrier" is meant to encompass any carrier, which does not interfere with the effectiveness of the biological activity of the ingredients and that is not toxic to the patient to whom it is administered. Examples of suitable pharmaceutical carriers are well known in the art and include phosphate buffered saline solutions, water, emulsions, such as oil/water emulsions, various types of wetting agents, sterile solutions etc. Such carriers can be formulated by conventional methods and can be administered to the subject at a suitable dose. Preferably, the compositions are sterile. These compositions may also contain adjuvants such as preservative, emulsifying agents and dispersing agents. Prevention of the action of microorganisms may be ensured by the inclusion of various antibacterial and antifungal agents. Administration of the suitable compositions may be effected by different ways, e.g. by intravenous, intraperetoneal, subcutaneous, intramuscular, topical or intradermal administration. The route of administration, of course, depends on the kind of therapy and the kind of compound contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, the kind of therapy, general health and other drugs being administered concurrently.

In another aspect of the invention the dimeric antigen-binding molecule as described herein above is used in the manufacture of a medicament for the treatment of B-cell malignancies (e.g. non-Hodgkin's lymphoma; chronic lymphocytic leukaemia; Hodgkin's lymphomia).

The methods for preparing pharmaceutical compositions, i.e. medicaments, and the clinical application of antigen binding molecules in the prevention and/or treatment of diseases such as, for example, cancer are known to the skilled artisan.

In a particular aspect of the invention the dimeric antigen binding molecule is bispecific and used for cancer therapy, because such antibodies can be used to retarget cytotoxic effector cells against tumor cells. This therapeutic concept is well known in the art. For example, clinical studies showed tumor regression in patients treated with an anti-CD3 x antitumor bispecific antibody (e.g. Canevari, S. et al., J. Natl. Cancer Inst., 87:1463-1469,1996) or patients treated with an anti-CD16 x antitumor bispecific antibody (e.g. Hartmann et al.; Clin Cancer Res. 2001;7(7):1873-81). Proof-of-concept has also been shown for various recombinant bispecific antibody molecules comprising only variable domains (Fv) such as, for example, dimeric and tetravalent CD3xCD19 antigen binding molecules having a domain order V_{H}A-V_{L}B-V_{H}B-V_{L}A (Cochlovius et al.; Cancer Research, 2000, 60:4336-4341)or recently in clinical studies with monomeric single-chain Fv antibody molecules of the BiTE®-format (two single-chain antibodies of different specificities linked together; Micromet AG, Germany; Bargou R. et al., Science, 2008, 321(5891):974-977; Baeuerle PA and Reinhardt C., Cancer Res. 2009, 69(12):4941-4944). The dimeric antigen binding molecules described herein can be used as medicaments and applied in methods of treatment in a similar way as the bispecific antibodies of the art, as they are capable of redirecting therapeutic, e.g. cytotoxic, mechanisms using the same combined antibody specificities.

The antigen-binding molecule and the compositions thereof can be in the form of an oral, intravenous, intraperitoneal, or other pharmaceutically acceptable dosage form. In some embodiments, the composition is administered orally and the dosage form is a tablet, capsule, caplet or other orally available form. In some embodiments, the composition is parenteral, e.g. intravenous, intraperitoneal, intramuscular, or subcutaneous, and is administered by means of a solution containing the antigen-binding molecule.

A skilled person will readily be able without undue burden to construct and obtain the antigen-binding molecules described herein by utilizing established techniques and standard methods known in the art, see for example Sambrook, Molecular Cloning A Laboratory Manual, Cold Spring Harbor Laboratory (1989) N.Y.; The Protein Protocols Handbook, edited by John M. Walker, Humana Press Inc. (2002); or Antibody engineering: methods and protocols / edited by Benny K.C. Lo; Benny K.C. II Series: Methods in molecular biology (Totowa, N.J.)). In addition, a skilled person will be able to make the antigen-binding molecules described herein by utilizing standard methods known in the art and modifying the methods described in US 7,129,330, Kipriyanov et al. J. Mol. Biol. (1999) 293, 41- 56 or Le Gall et al., 2004, Protein Engineering 17:357-366 such that dimeric antigen-binding molecules as described above comprising two polypeptide chains having the domain order V_{L}A-V_{H}B-V_{L}B-V_{H}A from the N-terminus to the C-terminus of each polypeptide chains are obtained.

The example below further illustrates the invention without limiting the scope of the invention.

### Example 1:

To construct functional dimeric tandem diabodies (TandAb^{®})using a domain arrangement other than V_{H}A-V_{L}B-V_{H}B-V_{L}A, several such dimeric tandem diabodies were constructed with the domain arrangement V_{L}A-V_{H}B-V_{L}B-V_{H}A according to the invention using the two domains of a humanized anti-CD19 single chain antibody and a humanized anti-CD3 single chain antibody, respectively. The findings were confirmed by using two variants of each antigen-binding molecule, representing the products of different stages of an affinity maturation procedure that was carried out for both the humanized anti-CD19 and humanized anti-CD3 antibodies.

The murine monoclonal antibodies HD37 and UCHT directed against CD19 and CD3, respectively, were the starting material for obtaining humanized antibodies with relatively high affinities. In each case the V_{H} domain was first combined with a library of human V_{L} in an scFv phagemid vector to select a suitable human V_{L} chain by phage display. In a second step the selected human V_{L} chain was combined with a library of VH domains in which the CDR3 region remained constant. This procedure resulted in a humanized anti CD19 and anti CD3, respectively, that only contained a short murine sequence in the VHCDR3 region. These clones were subsequently affinity matured introducing point mutations at residues thought to be involved in antigen binding. The best binding mutants were then selected by phage display. The clones chosen for constructing the TandAb were M13 and M39 binding to CD19 and C4 and LcHC21 binding to CD3.

The following antibodies according to the invention were generated:
Antibody A1: CD19^{M39}xCD3^{C4} (option 0) V_{H}^{CD3C4}-V_{L}^{CD19M39}-V_{H}^{CD19M39}-V_{L}^{CD3C4}
Antibody B: CD19^{M39}xCD3^{C4} (option 2) V_{H}^{CD3C4}-V_{H}^{CD19M39}-V_{L}^{CD19M39}-V_{H}^{CD3C4}
Antibody A2: CD19^{M13}xCD3^{LCHC21} (option 0) V_{H}^{CD3LCHC21}-V_{L}^{CD19M13}-V_{H}^{CD19M13}-V_{L}^{CD3LCHC21}
Antibody C: CD19^{M13}xCD3^{LCHC21}(option 2) V_{L}^{CD3LCHC21}-V_{H}^{CD19M13}-V_{L}^{CD19M13}-V_{H}^{CD3LCHC21}

The plasmids encoding the hybrid monomers V_{L}^{CD3C4}-V_{H}^{CD19M39}-V_{L}^{CD19M39}-V_{H}^{CD3C4} of antibody B and V_{L}^{D3LCHC21}-V_{H}^{CD19M13}-V_{L}^{D19M13}-V_{H}^{CD3LCHC21} of antibody C were generated by a DNA engineering and processing provider. The sequence backbone of the V_{L}^{CD3C4}-V_{H}^{CD19M39}-V_{L}^{CD19M39}-V_{H}^{CD3C4} monomer comprises the DNA sequences of two scFv antibodies, namely scFvCD19^{M39} and scFvCD3^{C4}, respectively. The V_{L}^{CD3LCHC21}-V_{H}^{CD19M13}-V_{L}^{CD19M13}-V_{H}^{CD3LCHC21} monomer sequence combines the variable domains of the single chain Fv CD19^{M13} and single chain Fv CD3^{LCHC21}. All four scFv were obtained by phage display selection of single chain antibodies against the antigens CD19 and CD3. In both cases the sequence information was used to construct the above hybrid monomers. A 9 amino acid (G₂S)₃ linker was used to link the domains with one another. The synthesized gene coding for V_{L}^{CD3C4}-V_{H}^{CD19M39}-V_{L}^{CD19M39}-V_{H}^{CD3C4} was cloned into the mammalian expression vector pCDNA5FRT (Invitrogen). The gene of V_{L}^{CD3LCHC21}-V_{H}^{CD19M13}-V_{L}^{CD19M13}-V_{H}^{CD3LCHC21} was also cloned into an expression vector and amplified by PCR using a forward primer introducing an NcoI cleaving site and a reverse primer introducing a NotI cleaving site. After analysis and isolation by agarose gel, the PCR product was subsequently double digested by NcoI and NotI and cloned into the NcoI and NotI linearised pSKK3 vector. The correct cloning was confirmed by DNA sequencing.

The vector map of pCDNA5FRT encoding antibody B is shown in Fig. 6. The vector map of pSKK3 encoding antibody C is shown in Fig. 7.

For high level production the vector containing the gene V_{L}^{CD3C4}-V_{H}^{CD19M39}-V_{L}^{CD19M39}-V_{H}^{CD3C4} was transiently transfected (using CaPO₄ into adherent HEK293 cells. Protein fermentation was performed under growth conditions well known in the art.

The recombinant protein was expressed as a His-Tag fusion protein with a signal peptide. The protein was isolated from cell culture supernatant by immobilized metal affinity chromatography (IMAC) as described (Kipriyanov et al., 1999, J.Mol.Biol., 293, 41-56). The purified material was subsequently analysed by SDS-PAGE. Coomassie staining of an SDS PAGE gel and size-exclusion chromatography on a calibrated Superdex 200 HR10/30 column (Amersham Pharmacia, Freiburg, Germany) in sodium-phosphate buffer (30mM NaPO₄ 0.75M arginine/HCl, pH6.0) revealed a pure and correctly assembled recombinant protein (Antibody B).

For high level expression, the gene coding for the humanized V_{L}^{CD3LCHC21}-V_{H}^{CD19M13}-V_{L}^{CD19M13}-V_{H}^{CD3LCHC21} monomer followed by a 6x His-Tag was cloned into the pSKK3 plasmid containing the hok/sok gene cell suicide system and a skp gene encoding the Skp/OmpH periplasmic factor (LeGall et al., 2004, J. Immunol. Methods, 285, 111-127). The plasmid was was transfected into an E.coli K12 strain (ATCC 31608™) .

The transformed bacteria were grown in shake flasks and induced essentially as described previously (Cochlovius et al., 2000, J. Immunol., 165, 888-895). The recombinant proteins were isolated from both the soluble periplasmic fraction and the bacterial medium supernatant by immobilized metal affinity chromatography (IMAC) as already described (Kipriyanov et al., 1999, J.Mol.Biol., 293, 41-56).

The purified material was subsequently analysed by SDS-PAGE stained by Coomassie blue and size-exclusion chromatography on a calibrated Superdex 200 HR10/30 column (Amersham Pharmacia, Freiburg, Germany) in sodium-phosphate buffer (30mM NaPO₄ 0.75M arginine/HCl, pH6.0). The product appeared to be pure and correctly assembled.

The comparative antibodies A1 and A2 were generated in the same way as antibodies B and C, respectively, wherein the domain order of antibodies A1 and A2, respectively, were reversed in comparison to that of antibodies B and C, respectively.

Cytotoxicity assays were performed essentially as described by T. Dreier et al. (2002, Int J Cancer 100, 690-697). The PMBCs that were used as effector cells were isolated from the peripheral blood of healthy volunteers by density gradient centrifugation. In some cases, the PBMC were cultured overnight in the presence of 25 U/mL human IL-2 before they were used as effector cells in the cytotoxicity assay. Purity and antigen expression of the isolated PBMC was checked by flow cytometry in each case (data not shown).

CD19⁺ JOK-1 or Raji target cells were cultured in RPMI 1640 medium supplemented with 10%FCS, 2 mM L-glutamine and 100 IU/mL penicillin G sodium and 100 µg/mL streptomycin sulfate (herein referred to as RPMI medium; all components from Invitrogen). For the cytotoxicity assay cells were labeled with 10 µM calcein AM (Molecular Probes/Invitrogen) for 30 min in RPMI medium without FCS at 37°C. After gently washing the labeled cells were resuspended in RPMI medium to a density of 1x10⁵/mL. 1x10⁴ target cells were then seeded together with 5x10⁵ PBMC with the indicated antibodies in individual wells of a roundbottom 96-well micro plate in a total volume of 200 µL/well. After centrifugation for 2 min at 200 g the assay was incubated for 4 hours at 37°C in a humidified atmosphere with 5% CO₂. 15 min prior to the end of incubation 20 µL of 10% Triton X-100 in RPMI medium were added to the wells with target cells only. 20 µL RPMI medium was added to all other wells. 100 µL cell culture supernatant were harvested from each well after an additional centrifugation for 5 min at 500 g, and the fluorescence of the released calcein was measured at 520 nm using a fluorescence plate reader (Victor 3, Perkin Elmer). On the basis of the measured counts, the specific cell lysis was calculated according to the following formula: [fluorescence (sample) - fluorescence (spontaneous)] / [fluorescence (maximum) - fluorescence (spontaneous)] x 100%. Fluorescence (spontaneous) represents the fluorescent counts from target cells in the absence of effector cells and antibodies and fluorescence (maximum) represents the total cell lysis induced by the addition of Triton X-100. Sigmoidal dose response curves and EC₅₀ values were calculated using the Prism software (GraphPad Software).

### Results:

The results of the cytotoxicity assays for tandem diabodies having the following domain order starting at the N-terminus of V_{H}A-V_{L}B-V_{H}B-V_{L}A (antibody A) and V_{L}A-V_{H}B-V_{L}B-V_{H}A (antibody B), respectively, using the anti CD19 variant M39 and the anti CD3 variant C4 are shown in Figure 3.

Surprisingly, there was a very large difference in the cytotoxic activity of the two tandem diabodies. The tandem diabody having the domain arrangement according to the invention designated as "antibody B" was more than 60x more active than the tandem diabody designated "antibody B" as determined by a comparison of their EC₅₀ values under the given conditions.

The superiority of the domain arrangement represented by the present invention (antibody C) for better cytotoxicity was confirmed by using two additional variants of the anti CD19 and anti CD3 antibodies (see Figure 4).

The EC₅₀ value of the tandem diabody with the domain order according to the invention represented by option 2 is extremely low (0.1pM). It is 27x more active than the TandAb represented by option 0 after comparing the EC50 values under the given conditions.

## Claims

1. A dimeric antigen-binding molecule specific for CD3 and CD19, composed of a first and a second polypeptide chain, each of the first and the second polypeptide chains comprising
- a first domain V_{L}A being a light chain variable domain specific for CD3;
- a second domain V_{H}B being a heavy chain variable domain specific for CD19;
- a third domain V_{L}B being a light chain variable domain specific for the CD19; and
- a fourth domain V_{H}A being a heavy chain variable domain specific for CD3,
wherein
- in the first and second polypeptide chain the first domain V_{L}A is linked with the second domain V_{H}B by a first linker L1, the second domain V_{H}B is linked with the third domain V_{L}B by a second linker L2 and the third domain V_{L}B is linked with the fourth domain V_{H}A by a third linker L3, wherein the linkers L1, L2 and L3 consist of 6, 7, 8, 9, 10, 11 or 12 amino acid residues,
- said domains are arranged in each of said first and second polypeptide chains in the order V_{L}A-V_{H}B-V_{L}B-V_{H}A from the N-terminus to the C-terminus of said polypeptide chains in an orientation preventing intramolecular pairing, and
- the first domain V_{L}A of the first polypeptide chain is in association with the fourth domain V_{H}A of the second polypeptide chain to form an antigen binding site for the first antigen A;
- the second domain V_{H}B of the first polypeptide chain is in association with the third domain V_{L}B of the second polypeptide chain to form an antigen binding site for the second antigen B;
- the third domain V_{L}B of the first polypeptide chain is in association with the second domain V_{H}B of the second polypeptide chain to form an antigen binding site for the second antigen B; and
- the fourth domain V_{H}A of the first polypeptide chain is in association with the first domain V_{L}A of the second polypeptide chain to form an antigen binding site for the first antigen A.

2. The antigen-binding molecule according to claim 1, wherein linkers L1, L2 and/or L3 consist of 9 contiguous amino acid residues.

3. The antigen-binding molecule according to any one of the claims 1 or 2, wherein the domains are human domains or humanized domains.

4. The antigen-binding molecule according to any one of the claims 1 to 3, wherein said antigen-binding molecule comprises at least one further functional unit.

5. A composition comprising the antigen-binding molecule according to any one of the claims 1 to 4 and a pharmaceutically acceptable carrier.

6. The antigen-binding molecule according to any one of the claims 1 to 4 for use as a medicament.

## Patentansprüche

1. Dimeres antigenbindendes Molekül, das für CD3 und CD 19 spezifisch ist, bestehend aus einer ersten und einer zweiten Polypeptidkette, wobei die erste und die zweite Polypeptidkette jeweils umfasst:
- eine erste Domäne V_{L}A, die eine leichtkettige variable Domäne ist, die für CD3 spezifisch ist;
- eine zweite Domäne V_{H}B, die eine schwerkettige variable Domäne ist, die für CD 19 spezifisch ist;
- eine dritte Domäne V_{L}B, die eine leichtkettige variable Domäne ist, die für CD19 spezifisch ist; und
- eine vierte Domäne V_{H}A, die eine schwerkettige variable Domäne ist, die für CD3 spezifisch ist,
wobei
- in der ersten und zweiten Polypeptidkette die erste Domäne V_{L}A mit der zweiten Domäne V_{H}B durch einen ersten Linker L1 verknüpft ist, die zweite Domäne V_{H}B mit der dritten Domäne V_{L}B durch einen zweiten Linker L2 verknüpft ist und die dritte Domäne V_{L}B mit der vierten Domäne V_{H}A durch einen dritten Linker L3 verknüpft ist, wobei die Linker L1, L2 und L3 aus 6, 7, 8, 9, 10, 11 oder 12 Aminosäureresten bestehen,
- die Domänen jeweils in der ersten und zweiten Polypeptidkette in der Reihenfolge V_{L}A-V_{H}B-V_{L}B-V_{H}A vom N-Terminus zum C-Terminus der Polypeptidketten in einer Ausrichtung angeordnet sind, die eine intramolekulare Paarung verhindert, und
- die erste Domäne V_{L}A der ersten Polypeptidkette in Verbindung mit der vierten Domäne V_{H}A der zweiten Polypeptidkette steht, um eine Antigenbindungsstelle für das erste Antigen A zu bilden;
- die zweite Domäne V_{H}B der ersten Polypeptidkette in Verbindung mit der dritten Domäne V_{L}B der zweiten Polypeptidkette steht, um eine Antigenbindungsstelle für das zweite Antigen B zu bilden;
- die dritte Domäne V_{L}B der ersten Polypeptidkette in Verbindung mit der zweiten Domäne V_{H}B der zweiten Polypeptidkette steht, um eine Antigenbindungsstelle für das zweite Antigen B zu bilden; und
- die vierte Domäne V_{H}A der ersten Polypeptidkette in Verbindung mit der ersten Domäne V_{L}A der zweiten Polypeptidkette steht, um eine Antigenbindungsstelle für das erste Antigen A zu bilden.

2. Antigenbindendes Molekül nach Anspruch 1, wobei die Linker L1, L2 und/oder L3 aus 9 benachbarten Aminosäureresten bestehen.

3. Antigenbindendes Molekül nach einem der Ansprüche 1 oder 2, wobei die Domänen menschliche Domänen oder humanisierte Domänen sind.

4. Antigenbindendes Molekül nach einem der Ansprüche 1 bis 3, wobei das antigenbindende Molekül mindestens eine weitere funktionelle Einheit umfasst.

5. Zusammensetzung, umfassend das antigenbindende Molekül nach einem der Ansprüche 1 bis 4 und einen pharmazeutisch annehmbaren Träger.

6. Antigenbindendes Molekül nach einem der Ansprüche 1 bis 4 zur Verwendung als Medikament.

## Revendications

1. Molécule à liaison d'antigène dimère spécifique pour CD3 et CD19, composée d'une première et d'une deuxième chaîne polypeptidique, chacune des première et deuxième chaînes polypeptidiques comprenant
- un premier domaine V_{L}A qui est un domaine variable de chaîne légère spécifique pour CD3;
- un deuxième domaine V_{H}B qui est un domaine variable de chaîne lourde spécifique pour CD 19;
- un troisième domaine V_{L}B qui est un domaine variable de chaîne légère spécifique pour CD 19; et
- un quatrième domaine V_{H}A qui est un domaine variable de chaîne lourde spécifique pour CD3,
dans laquelle
- dans la première et la deuxième chaîne polypeptidique, le premier domaine V_{L}A est relié au deuxième domaine V_{H}B par un premier lieur L1, le deuxième domaine V_{H}B est lié au troisième domaine V_{L}B par un deuxième lieur L2 et le troisième domaine V_{L}B est lié au quatrième domaine V_{H}A par un troisième lieur L3, où les lieurs L1, L2 et L3 consistent en 6, 7, 8, 9, 10, 11 ou 12 résidus d'acide aminé,
- lesdits domaines sont disposés dans chacune desdites première et deuxième chaînes polypeptidiques dans l'ordre V_{L}A-V_{H}B-V_{L}B-V_{H}A de l'extrémité N-terminale à l'extrémité C-terminale desdites chaînes polypeptidiques selon une orientation qui empêche le couplage intramoléculaire, et
- le premier domaine de V_{L}A de la première chaîne polypeptidique est en association avec le quatrième domaine V_{H}A de la deuxième chaîne polypeptidique, pour former un site de liaison d'antigène pour le premier antigène A;
- le deuxième domaine V_{H}B de la première chaîne polypeptidique est en association avec le troisième domaine V_{L}B de la deuxième chaîne polypeptidique, pour former un site de liaison d'antigène pour le deuxième antigène B;
- le troisième domaine V_{L}B de la première chaîne polypeptidique est en association avec le deuxième domaine V_{H}B de la deuxième chaîne polypeptidique, pour former un site de liaison d'antigène pour le deuxième antigène B; et
- le quatrième domaine V_{H}A de la première chaîne polypeptidique est en association avec le premier domaine V_{L}A de la deuxième chaîne polypeptidique, pour former un site de liaison d'antigène pour le premier antigène A.

2. Molécule à liaison d'antigène selon la revendication 1, dans laquelle les lieurs L1, L2 et/ou L3 consistent en 9 résidus d'acide aminé contigus.

3. Molécule à liaison d'antigène selon l'une quelconque des revendications 1 ou 2, dans laquelle les domaines sont des domaines humains ou des domaines humanisées.

4. Molécule à liaison d'antigène selon l'une quelconque des revendications 1 à 3, dans laquelle ladite molécule à liaison d'antigène comprend au moins une autre unité fonctionnelle.

5. Composition comprenant la molécule à liaison d'antigène selon l'une quelconque des revendications 1 à 4 et un support pharmaceutiquement acceptable.

6. Molécule à liaison d'antigène selon l'une quelconque des revendications 1 à 4 destinée à être utilisée comme médicament.
